(19) Europäisches Patentamt / European Patent Office / Office européen des brevets

(11) **EP 2 566 573 B1**

(12) **FASCICULE DE BREVET EUROPEEN**

(45) Date de publication et mention
de la délivrance du brevet:
**29.07.2015 Bulletin 2015/31**

(21) Numéro de dépôt: **11723540.8**

(22) Date de dépôt: **29.04.2011**

(51) Int Cl.:
**A61N 1/02** (2006.01)

(86) Numéro de dépôt international:
**PCT/FR2011/050984**

(87) Numéro de publication internationale:
**WO 2011/135273 (03.11.2011 Gazette 2011/44)**

(54) **IMPLANT A GEOMETRIE TRIDIMENSIONNELLE POUR LA STIMULATION ELECTRIQUE D'UNE STRUCTURE NERVEUSE**

IMPLANTAT MIT DREIDIMENSIONALER FORM ZUR ELEKTRISCHEN STIMULATION EINER NERVENSTRUKTUR

IMPLANT HAVING THREE-DIMENSIONAL SHAPE FOR ELECTRICALLY STIMULATING A NERVE STRUCTURE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.04.2010 FR 1053381**

(43) Date de publication de la demande:
**13.03.2013 Bulletin 2013/11**

(73) Titulaire: **Université Pierre et Marie Curie (Paris 6)
75005 Paris (FR)**

(72) Inventeurs:
• **LORACH, Henri**
  **F-75005 Paris (FR)**
• **DJILAS, Milan**
  **F-75012 Paris (FR)**
• **YVERT, Blaise**
  **F-33360 Carignan de Bordeaux (FR)**
• **BERGONZO, Philippe**
  **F-91300 Massy (FR)**
• **LISSORGUES, Gaelle**
  **F-94170 Le Perreux sur Marne (FR)**
• **ROUSSEAU, Lionel**
  **F-94170 Le Perreux sur Marne (FR)**
• **BENOSMAN, Ryad, Benjamin**
  **F-75010 Paris (FR)**
• **PICAUD, Serge**
  **F-77210 Avon (FR)**
• **SAHEL, José**
  **F-75013 Paris (FR)**
• **IENG, Siohoi**
  **F-75013 Paris (FR)**

(74) Mandataire: **Cabinet Plasseraud
52, rue de la Victoire
75440 Paris Cedex 09 (FR)**

(56) Documents cités:
**WO-A2-2004/073547    WO-A2-2009/006636
US-A- 6 112 124    US-A1- 2008 077 196**

• **ROUSSEAU L ET AL: "Microfabrication of new microelectrode arrays equipped with a ground surface configuration for focal neural microstimulation", JOURNAL OF MICROMECHANICS AND MICROENGINEERING IOP PUBLISHING LTD. UK, vol. 19, no. 7, juillet 2009 (2009-07), XP002595978, ISSN: 0960-1317**

EP 2 566 573 B1

**EP 2 566 573 B1**

**Description**

La présente invention concerne des implants utilisables pour stimuler électriquement une structure nerveuse.

Pour plusieurs pathologies et handicaps moteurs ou sensoriels, la stimulation électrique de structures nerveuses a été proposée voire validée au niveau clinique. Il s'agit par exemple de la stimulation électrique à haute fréquence pour le traitement de la maladie de Parkinson, la stimulation de l'oreille interne pour le traitement de la surdité, et plus récemment de la rétine ou du cortex visuel pour le traitement de la cécité. Cependant, les applications envisageables sont très nombreuses, notamment pour le contrôle des sphincters, le traitement de l'épilepsie et d'autres pathologies neurologiques.

Pour mettre en oeuvre ce type de stimulation, un implant est amené au contact de la structure nerveuse concernée. Un tel implant possède des électrodes par lesquelles une différence de potentiel électrique est appliquée, ou un courant est injecté, afin de stimuler des cellules nerveuses. Plusieurs configurations d'électrodes ont été proposées pour obtenir une stimulation efficace de la structure visée.

Dans la configuration dite monopolaire, le courant s'écoule entre une électrode de stimulation et une électrode de retour éloignée (à l'infini). Cette configuration monopolaire fournit une stimulation de sélectivité spatiale médiocre. Or la sélectivité spatiale de la stimulation électrique est une propriété recherchée dans beaucoup d'applications. Par exemple, si l'implant comporte un réseau d'unités microscopiques juxtaposées dans le but d'effectuer des stimulations électriques indépendantes les unes des autres notamment pour communiquer une image nette à des neurones de la rétine ou du cortex visuel, il importe de fournir des stimulations électriques bien localisées à chacune de ces unités ou pixel, avec des fuites électriques ("cross-talk") aussi réduites que possible entre pixels adjacents.

Des configurations bipolaires utilisent une paire d'électrodes pour chaque zone à stimuler de la structure nerveuse, excitées par des potentiels électriques positifs et négatifs. La localisation de la stimulation électrique est améliorée par rapport à la configuration monopolaire, mais peut être encore insuffisante pour certaines applications.

En pratique, des configurations d'électrodes avec plan de masse sont préférables aux électrodes bipolaires puisque l'électrode de retour est alors commune à toutes les unités de l'implant, divisant ainsi par deux le câblage interne du système. Dans "Improved Focalization of Electrical Microstimulation Using Microelectrode Arrays: A Modeling Study" (PLoS ONE, www.plosone.org, Vol. 4, n° 3, e4828, mars 2009), S. Joucla et B. Yvert ont montré une focalisation améliorée de la microstimulation avec un composant dont la surface présente un plan de masse coplanaire avec des électrodes de stimulation.

Dans "Migration of retinal cells through a perforated membrane: implications for a high-resolution prosthesis" (Investigative Ophthalmology & Visual Science, septembre 2004, Vol. 45, n°9, pages 3266-3270), D. Palanker, et al. ont étudié la capacité de cellules rétiniennes de rat à migrer à travers une membrane perforée électriquement inerte, et imaginé un implant à configuration tridimensionnelle avec des électrodes en saillie au-dessus d'une membrane. Mais un tel implant, également décrit dans WO 2004/073547, paraît difficilement réalisable en pratique.

Dans "Microfabrication of new microelectrode arrays equipped with a ground surface configuration for focal neural microstimulation", Journal of Micromechanics and Microengineering, Vol. 19, No. 7, page 074010, juin 2009, D. Rousseau, et al. décrivent un implant à structure essentiellement bidimensionnelle, à la surface duquel une électrode de masse alterne avec ces électrodes en or.

De façon générale, il est souhaitable de produire des stimulations localisées et capables de remplir leur rôle de stimulation sans endommager les tissus. Des études cliniques ont montré que les intensités de courant permettant d'obtenir une réponse dans les neurones ciblés peuvent excéder les seuils de sécurité pour les tissus. De plus, des applications comme la vision imposent de multiplier le nombre d'électrodes pour une même taille totale de l'implant et donc d'en augmenter la résolution spatiale.

Il existe donc un besoin de concevoir des structures d'électrodes qui permettent d'augmenter la focalisation des stimulations tout en limitant l'amplitude des courants délivrés.

Il est proposé un implant pour la stimulation électrique d'une structure nerveuse, comprenant:

- un substrat électriquement isolant;
- un réseau de cavités formées dans une surface supérieure du substrat, les cavités ayant une profondeur supérieure à 15 μm;
- des électrodes de stimulation, chacune disposée au fond de l'une des cavités; et
- une couche électriquement conductrice formant un plan de masse en partie supérieure des cavités.

L'implant sera installé en mettant la surface supérieure du substrat, munie du plan de masse, au contact d'un tissu comportant des cellules nerveuses à stimuler, qui peuvent se situer à des profondeurs plus ou moins grandes, généralement d'une à quelques dizaines de microns (μm).

La configuration tridimensionnelle de l'implant avec plan de masse permet de focaliser la stimulation électrique des cellules cibles à l'intérieur de la cavité. On assure donc une grande sélectivité spatiale qui permet d'atteindre un niveau de stimulation donné dans la zone cible avec un courant total réduit, ce qui minimise les dommages aux tissus traités. Un autre avantage de la sélectivité obtenue est de permettre d'augmenter le nombre d'unités de stimulation de l'implant tout en commandant ces unités indépendamment les unes des autres si l'application le requiert.

Dans un mode de réalisation de l'implant, chaque cavité a une forme évasée s'élargissant du fond de la cavité vers la surface supérieure du substrat. Ceci facilite la pénétration et la répartition des cellules dans les cavités.

La profondeur des cavités est choisie en fonction de la profondeur à laquelle les cellules cibles sont supposées se trouver dans le tissu à stimuler. Souvent, une couche de cellules gliales se développe à l'interface entre le tissu et l'électrode et on cherche à stimuler des neurones situés derrière cette couche. Des simulations ont montré que, pour les applications typiques, des cavités ou puits de profondeur supérieure à 15 $\mu$m permettaient de bien focaliser la stimulation électrique sur la zone cible.

Des cavités de trop grande profondeur ne sont pas souhaitables non plus, notamment lorsque leurs parois latérales sont inclinées, car cela limite la densité de cavités sur le substrat. On pourra en particulier garder une profondeur de cavité inférieure à 50 $\mu$m. Des cavités de profondeur comprise entre 25 et 35 $\mu$m paraissent optimales pour le compromis entre sélectivité et taille des cavités.

Pour limiter la taille des cavités, chaque électrode de stimulation peut avoir, sur la surface de fond de sa cavité respective, une dimension inférieure à 60 $\mu$m.

D'autres paramètres peuvent être ajustés pour optimiser les performances de l'implant. Dans une configuration, chaque cavité a une surface de fond isolante recouverte de manière partielle par une électrode de stimulation. On peut alors favoriser la stimulation de cellules relativement proches de la surface du tissu à traiter.

Dans une autre configuration, chaque électrode de stimulation a une partie centrale s'étendant sur une surface de fond de sa cavité respective et une partie périphérique débordant sur les parois latérales de ladite cavité. La partie centrale de l'électrode de stimulation a avantageusement une dimension (diamètre ou coté) inférieure à 40 $\mu$m, permettant de maximiser la focalisation de la stimulation électrique.

Une autre possibilité est de jouer sur la forme de la couche électriquement conductrice formant le plan de masse. On peut notamment la former de façon qu'elle comporte, pour chaque cavité du réseau, une partie débordant sur les parois latérales de cette cavité.

D'autres particularités et avantages de la présente invention apparaîtront dans la description ci-après d'exemples de réalisation non limitatifs, en référence aux dessins annexés, dans lesquels :

- la figure 1 est un schéma en perspective d'un exemple d'implant selon l'invention;
- la figure 2 est une vue schématique en coupe d'une cavité de l'implant de la figure 1;
- les figures 3 et 4 sont des vues semblables à celle de la figure 2 concernant d'autres réalisations possibles d'une cavité de l'implant;
- les figures 5 et 6 sont des graphiques montrant des résultats obtenus en simulant le comportement de cavités selon les figures 3 et 4, respectivement, avec différentes valeurs de paramètres dimensionnels; et
- les figures 7 et 8 sont des graphiques montrant l'effet d'une perturbation de certains paramètres sur les résultats de simulation représentés sur les figures 5 et 6.

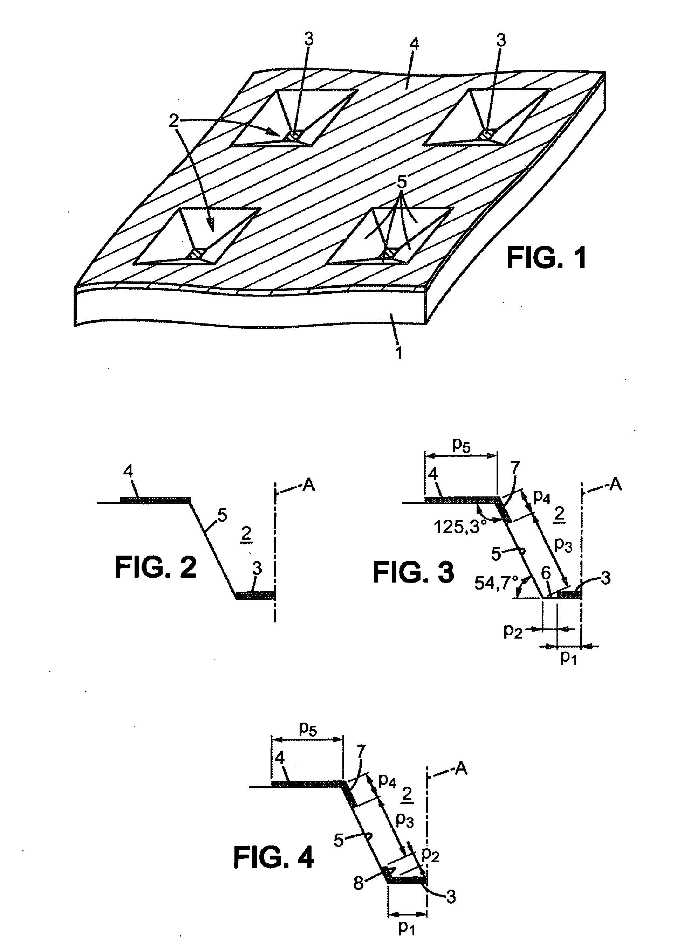

Un exemple de réalisation d'un implant selon l'invention est illustré par les figures 1 et 2. Il comprend un substrat électriquement isolant 1 dont la surface supérieure présente un réseau de cavités 2. Une électrode de stimulation 3 se trouve au fond de chaque cavité 2.

Une électrode de retour 4 commune aux différentes cavités, ou plan de masse, est constituée par une couche électriquement conductrice qui, dans l'exemple des figures 1 et 2, couvre sensiblement toute la surface supérieure du substrat 1 en dehors des cavités 2. Entre cette couche conductrice 4 et les électrodes de stimulation 3, les cavités 2 ont une paroi latérale 5 dépourvue de matériau électriquement conducteur.

L'application d'une différence de potentiel, ou l'injection d'un courant, entre une électrode de stimulation 3 et le plan de masse 4 fait propager du courant dans le milieu qui se trouve à l'intérieur de la cavité 2. Les cavités 2 et les électrodes 3, 4 peuvent être dimensionnées de manière à focaliser le champ électrique, ou la densité de courant, dans les cavités 2 en minimisant sa propagation dans le milieu environnant.

Dans l'exemple des figures 1 et 2, les cavités 2 ont une forme générale de pyramide inversée et à sommet tronqué. Chaque cavité 2 a ainsi un fond plat parallèle à la surface supérieure du substrat 1. Ce fond plat est totalement recouvert par l'électrode de stimulation 3 dans cet exemple.

Il peut être également envisagé des cavités de formes et de profils différents, en modifiant le dessin de la structure de départ, par exemple ronde, triangulaire, hexagonale, octogonale, etc.

D'autres configurations d'électrodes, où la géométrie générale de la cavité reste semblable à celle des figures 1 et 2, sont illustrées par les figures 3 et 4.

Dans le cas de la figure 3, l'électrode de stimulation 3 ne couvre pas tout le fond de la cavité 2. Elle est entourée par une bordure isolante 6 entre les bords de l'électrode de stimulation 3 et la paroi latérale 5 de la cavité. On note $p_1$

la distance entre l'axe de symétrie A de la cavité et le bord de l'électrode de stimulation 3, et $p_2$ la distance entre le bord de l'électrode de stimulation 3 et la paroi latérale 5 de la cavité 2. L'électrode de stimulation 3 a donc pour dimension 2×p1 suivant deux directions perpendiculaires dans le plan parallèle au substrat 1, et est entourée par l'anneau isolant 6 de largeur $p_2$. La couche conductrice du plan de masse 4 s'étend en partie dans le plan de la surface supérieure du substrat 1, sur une largeur $p_5$ autour de chaque cavité, et déborde sur les parois latérales 5 de chaque cavité, en formant à la partie supérieure de chaque paroi 5 une bande conductrice 7 de largeur $p_4$. On note $p_3$ la largeur de la partie isolante de la paroi latérale 5 de la cavité 2, entre l'extrémité inférieure de la bande conductrice 7 et le fond de la cavité 2.

Dans le cas de la figure 4, le plan de masse 4 se présente comme dans le cas de la figure 3, avec les paramètres dimensionnels $p_4$ et $p_5$ précités. Cependant, l'électrode de stimulation 3 couvre tout le fond de la cavité 2, de dimension 2×$p_1$, et déborde dans une partie périphérique 8 sur les parois latérales 5 de la cavité 2. Cette partie périphérique 8 a, le long de la paroi 5, une largeur notée $p_2$ dans ce cas de figure. La largeur $p_3$ de la partie isolante de la paroi latérale 5 de la cavité 2. est alors mesurée entre l'extrémité inférieure de la bande conductrice 7 et l'extrémité supérieure de la partie périphérique 8 de l'électrode de stimulation 3. L'exemple dessiné sur les figures 1 et 2 est un cas limite de la figure 3 ou 4, où $p_2 = p_4 = 0$.

L'implant est destiné à être appliqué *in vivo* contre un tissu nerveux, en présentant sa surface supérieure revêtue du plan de masse 4 en face du tissu. Le fait que les cavités 2 aient une forme évasée s'élargissant du fond de la cavité vers la surface supérieure du substrat 1 facilite la pénétration des cellules nerveuses à l'intérieur de ces cavités. À titre d'exemple, les parois latérales 5 qui délimitent transversalement une cavité 2 forment un angle de 125,3° avec le plan de la surface supérieure du substrat 1. En d'autres termes, l'angle d'inclinaison de la paroi latérale 5 de la cavité 2 est de 54,7°, ce qui correspond à l'angle de gravure préférentiel dans une surface de silicium cristallin d'orientation cristallographique (100).

Des simulations ont été effectuées avec des cavités de sections transversales conformes aux figures 3 et 4 en considérant que les cavités 2 avaient une forme à symétrie de révolution autour de leur axe A. Le modèle physique utilisé dans les simulations était un modèle bidimensionnel à symétrie de révolution de milieu conducteur sous courants continus, défini par les équations de Maxwell:

$$J = \sigma.E$$

$$\nabla J = Q$$

$$Q = -\nabla(\sigma.\nabla V),$$

où J est le vecteur densité de courant, E est le vecteur champ électrique, $\sigma$ est la conductivité électrique du milieu, Q est la charge électrique et V est le potentiel électrique.

Dans ces simulations, les conditions aux limites suivantes étaient imposées par les caractéristiques des matériaux et de la stimulation. Pour les segments (vus en coupe transversale) constituant l'électrode de stimulation 3, l'écoulement du courant était vers l'intérieur avec une densité de courant correspondant à l'intensité, fixée à 10 $\mu$A, divisée par l'aire totale de l'électrode de stimulation. Afin de ne pas surestimer la sélectivité, l'électrode de retour (ou plan de masse) n'était pas modélisée comme une masse idéale, mais comme une résistance distribuée à potentiel nul (conductivité de 338 S/m). A part ces parties conductrices, les autres parties du modèle étaient définies comme des isolants électriques. Les distributions de densité de courant ont été calculées dans un domaine rectangulaire D = [0, 0] × [300 $\mu$m, 600 $\mu$m]. La résistivité. électrique de ce domaine était fixée à 50 $\Omega$.m (approximation de la résistivité des couches restantes dans une rétine dégénérée, dont les photorécepteurs ne sont plus fonctionnels).

Les géométries d'électrodes ont été optimisées pour trouver les paramètres optimaux du modèle qui fournissent la meilleure sélectivité de la stimulation. Une géométrie d'électrode était considérée optimale si son jeu de paramètres produisait la plus forte concentration de courant dans une zone cible définie comme un rectangle T = demi-largeur [de 0 à 20 $\mu$m] × hauteur dans la cavité [de 20 $\mu$m à 40 $\mu$m]. Les dimensions de cette zone cible ont été choisies pour correspondre approximativement à la localisation des cellules fonctionnelles cibles, en prenant en compte une mince couche isolante de tissu fibreux entre les électrodes et le tissu rétinien.

La sélectivité était quantifiée dans ces simulations en divisant l'intégrale de surface de la distribution de densité de courant dans la zone cible par l'intégrale de surface de la distribution de densité de courant en dehors de cette zone cible. À des fins de comparaison, l'optimisation a en outre été effectuée dans le cas d'une structure planaire (semblable à la configuration de la figure 3, mais avec $p_3 = p_4 = 0$.

Les plages des paramètres et leurs pas d'incrémentation entre les itérations utilisées dans l'optimisation sont indiquées au Tableau I, où les valeurs relatives aux segments horizontaux ($p_1$, $p_2$ et $p_5$ dans le cas de la figure 3; $p_1$ et $p_5$ dans le cas de la figure 4) représentent les longueurs de segments, tandis que les valeurs relatives aux segments inclinés ($p_3$ et $p_4$ dans le cas de la figure 3; $p_2$, $p_3$ et $p_4$ dans le cas de la figure 4) représentent les longueurs de leurs projections le long de l'axe A. L'optimisation était réalisée sous les contraintes supplémentaires que (1) les ouvertures des masques de fabrication correspondant aux surfaces actives d'électrodes doivent être espacées mutuellement d'au moins 5 μm et (2) la profondeur de cavité ne dépasse pas 50 μm.

Tableau I

|  |  | $P_1$ [μm] | $p_2$ [μm] | $p_3$ [μm] | $p_4$ [μm] | $p_5$ [μm] |
|---|---|---|---|---|---|---|
| Planaire | Valeur initiale | 5 | 5 | 0 | 0 | 5 |
|  | Pas | 5 | 5 | - | - | 5 |
|  | Valeur finale | 50 | 50 | - | - | 30 |
| Fig. 3 | Valeur initiale | 5 | 0 | 0 | 0 | 5 |
|  | Pas | 5 | 2 | 2 | 2 | 5 |
|  | Valeur finale | 50 | 10 | 50 | 50 | 30 |
| Fig. 4 | Valeur initiale | 0 | 0 | 0 | 0 | 5 |
|  | Pas | 5 | 2 | 2 | 2 | 5 |
|  | Valeur finale | 50 | 50 | 50 | 50 | 30 |

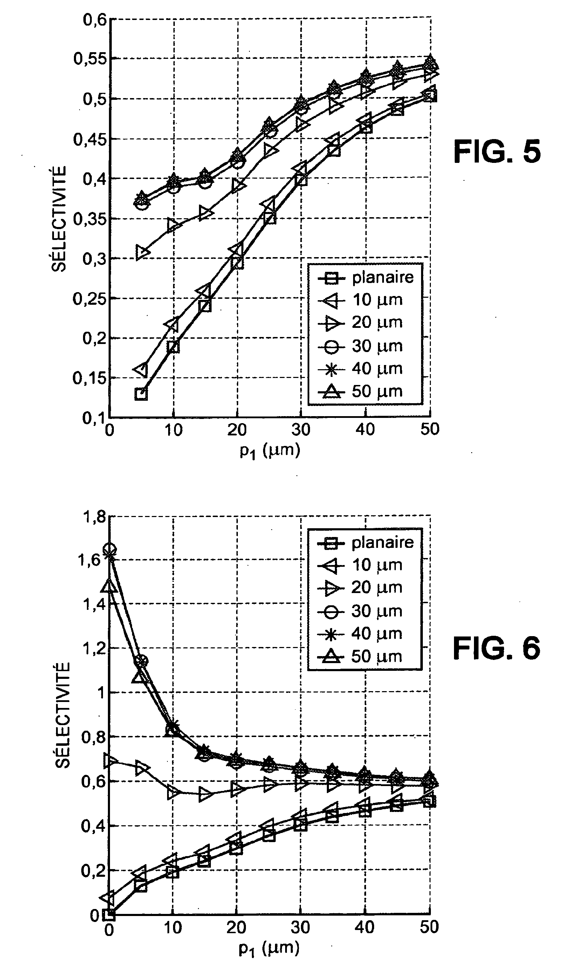

Pour les cas particuliers des géométries des figures 3 et 4, les paramètres optimaux en fonction de la profondeur de la cavité sont résumés dans le Tableau II. Dans le cas de la figure 3, la profondeur de cavité est la somme des projections sur l'axe A des paramètres $p_3$ et $p_4$, tandis que dans le cas de la figure 4, c'est la somme des projections sur l'axe A des paramètres $p_2$, $p_3$ et $p_4$. La sélectivité des électrodes en fonction du paramètre $p_1$ représentant la demi-dimension de l'électrode de stimulation 3 et de la profondeur de la cavité 2 est représentée sur la figure 5 pour la configuration d'électrodes de la figure 3 et sur la figure 6 pour la configuration d'électrodes de la figure 4. Sur ces figures 5 et 6, chaque point a été obtenu en trouvant les valeurs optimales des paramètres $p_2$, $p_3$, $p_4$ et $p_5$ pour des valeurs donnée de $p_1$ et de la profondeur de cavité.

Tableau II

|  | Profondeur cavité [μm] | $p_1$ [μm] | $p_2$ [μm] | $p_3$ [μm] | $p_4$ [μm] | $p_5$ [μm] | Sélectivité |
|---|---|---|---|---|---|---|---|
| Fig. 3 | 10 | 50 | 0 | 7 | 0 | 30 | 0,50 |
|  | 20 | 50 | 0 | 14 | 0 | 30 | 0,53 |
|  | 30 | 50 | 0 | 21 | 0 | 30 | 0,54 |
|  | 40 | 50 | 0 | 28 | 0 | 10 | 0,54 |
|  | 50 | 50 | 0 | 31 | 4 | 5 | 0,54 |
| Fig. 4 | 10 | 50 | 1 | 6 | 0 | 30 | 0,51 |
|  | 20 | 0 | 9 | 6 | 0 | 30 | 0,69 |
|  | 30 | 0 | 16 | 6 | 0 | 5 | 1,65 |
|  | 40 | 0 | 18 | 6 | 4 | 5 | 1,62 |
|  | 50 | 0 | 18 | 7 | 10 | 5 | 1,47 |

Dans la configuration selon la figure 3, la sélectivité augmente avec le paramètre $p_1$, tandis qu'elle décroît dans la configuration selon la figure 4. Dans les deux cas, la sélectivité s'améliore lorsque la cavité 2 devient plus profonde. Pour une profondeur de cavité inférieure à 20 μm (bord inférieur de la zone cible), il y a peu de différence entre les deux configurations, et avec la configuration planaire, même si une certaine amélioration est déjà notée pour une profondeur de 10 μm. On peut se donner 15 μm comme valeur minimale de la profondeur pour la conception d'un implant.

Pour les plus grandes profondeurs, la configuration de la figure 4 procure les meilleurs résultats, mais on observe déjà une amélioration sensible avec la configuration de la figure 3 par rapport au cas planaire. Pour chaque configuration tridimensionnelle des électrodes, la sélectivité atteint son maximum pour une profondeur de l'ordre de 30 μm. Cependant, on n'observe pas de dégradation sensible de la sélectivité lorsque la profondeur augmente vers 50 μm. Le compromis entre sélectivité et encombrement est le plus satisfaisant pour les profondeurs de cavité comprises entre 25 et 35 μm.

Les paramètres optimaux de la configuration de la figure 4 procurent la meilleure sélectivité pour les plus petites tailles d'électrode de stimulation. Ainsi, pour une électrode de diamètre 10 $\mu$m (p$_1$ = 5 $\mu$m), il y a, par rapport à la configuration planaire, une augmentation de la sélectivité de l'ordre de trois pour la configuration de la figure 3 et de l'ordre de dix pour celle de la figure 4.

De façon générale, une électrode de stimulation 3 de dimension inférieure à 60 $\mu$m sur la surface de fond de la cavité 2 (soit p$_1$ < 30 $\mu$m) a pour avantage de procurer des cavités 2 relativement compactes, donc réalisables avec une densité relativement élevée sur le substrat. Dans le cas de la figure 4, où l'électrode de stimulation 3 déborde dans sa partie périphérique 8 sur la paroi latérale de la cavité, les très petites tailles d'électrode de stimulation 3 (moins de 40 $\mu$m, soit p$_1$ < 20 $\mu$m) ont en outre l'avantage remarquable de donner lieu à des valeurs très élevées de la sélectivité comme le montre la figure 6.

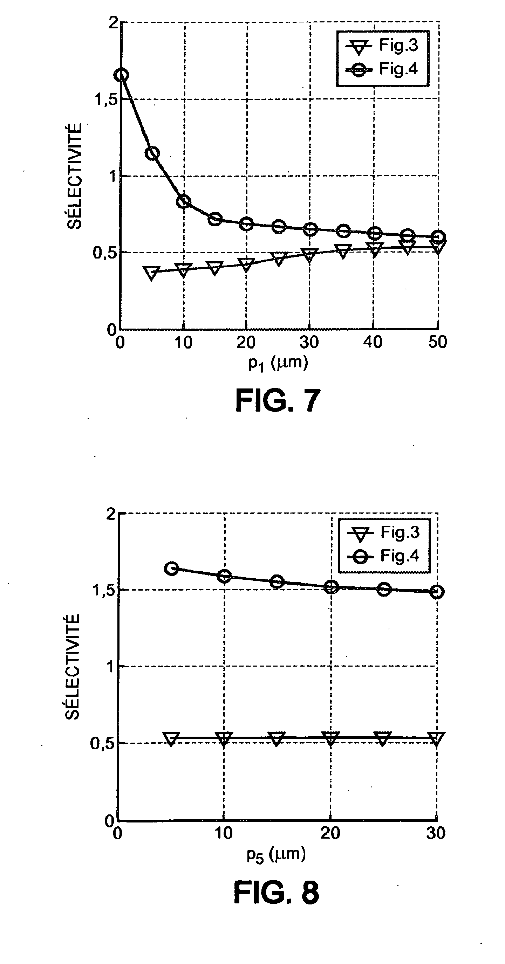

Pour regrouper autant d'électrodes que possible dans un réseau, il convient de prendre les paramètres p$_1$ et p$_5$ aussi petits que possible car cela conduit à une taille d'électrode minimale et donc à la distance inter-électrodes la plus petite. L'effet d'une perturbation de ces deux paramètres p$_1$, p$_5$ a été étudié et résumé sur les figures 7 et 8. Pour cela, on a fait varier le paramètre p$_1$ dans la plage indiquée au Tableau I en maintenant les quatre autres paramètres à leurs valeurs optimales trouvées pour une profondeur de cavité de 30 $\mu$m. La même procédure a été répétée pour le paramètre p$_5$.

Comme le montre la figure 7, changer la taille de l'électrode de stimulation dans la configuration de la figure 3 a relativement peu d'influence sur la sélectivité. En revanche, la sélectivité se dégrade quand p$_1$ augmente dans la configuration de la figure 4. Dans ce dernier cas, il est donc intéressant de choisir p$_1$ aussi petit que possible, en ayant soin que les densité de courant produites restent en-deçà d'une limite de sécurité pour le matériau de l'électrode. Le platine peut délivrer sans problème de 0,35 à 0,40 mC/cm$^2$ et l'oxyde d'iridium a quant à lui une limite de stimulation en sécurité allant jusqu'à 3 à 4 mC/cm$^2$. Les petites tailles d'électrode de stimulation ont aussi un avantage en termes de réduction du seuil d'activation des cellules. Cependant, un problème potentiel si la valeur de p$_1$ devient trop petite est que la cavité peut devenir trop étroite. Une cellule bipolaire rétinienne a une taille de l'ordre de 10 $\mu$m de sorte que le tissu rétinien qu'on cherche à stimuler risque de ne pas pénétrer dans la cavité 2 si celle-ci devient trop petite.

Le peu d'effet sur la sélectivité d'une perturbation du paramètre p$_5$ (figure 8) signifie que le courant de stimulation reste toujours bien confiné à l'intérieur de la cavité. Une extension très réduite de l'électrode de retour sur la surface supérieure du substrat 1 suffit à éliminer les fuites de courant inter-électrodes ("cross-talk"). Les cavités 2 peuvent donc être réunies sur le substrat 1 quasiment bord-à-bord. En combinaison avec le choix d'une petite valeur de p$_1$, ceci permet la conception de réseaux d'électrodes de haute densité. Le nombre maximal d'électrodes est finalement contraint par la capacité d'implanter des pistes conductrices pour les alimenter, ces pistes devant avoir une largeur minimum à prendre en considération.

Les simulations rapportées ci-dessus montrent que les structures d'électrodes tridimensionnelles proposées améliorent la focalisation de la stimulation dans un implant destiné à la rétine ou à d'autres structures nerveuses, appartenant au système nerveux central ou au système nerveux périphérique.

Les technologies de l'industrie du silicium sont utilisables pour fabriquer un implant ayant les caractéristiques ci-dessus. Une tranche de silicium monocristallin est alors utilisée comme moule. On forme par exemple des pyramides tronquées sur une surface (100) de cette tranche par un procédé de gravure humide à travers un masque ayant des motifs reproduisant la forme des fonds de cavité. La gravure s'opère préférentiellement suivant les plans (111), ce qui laisse l'angle de 54,7° pour les parois latérales 5 comme mentionné ci-dessus, des décrochements pouvant être présents près des angles des pyramides. Une couche conductrice, par exemple en platine ou en oxyde d'iridium, est déposée à travers un masque de photorésist de forme appropriée afin de réaliser le plan de masse 4 et les électrodes de stimulation 3 au fond des cavités 2 (c'est-à-dire au sommet des pyramides tronquées). Une résine d'un polymère biocompatible, par exemple un polyimide ou un parylène, est déposée sur la structure pour recouvrir les pyramides et on la fait polymériser pour constituer le substrat 1. Les connexions des électrodes sont réalisées sur ce substrat, puis le silicium est enlevé (par exemple par oxydation puis gravure chimique) pour obtenir l'implant dont la surface supérieure est revêtue du plan de masse 4 et présente les cavités 2 de formes et dimensions souhaitées.

Les technologies du silicium offrent également l'avantage de pouvoir réaliser des gravures de type isotrope, soit par gravure plasma ou liquide. Ceci permet d'obtenir un assez large éventail de formes pour les cavités. En partant d'un motif cercle, on peut obtenir une cavité en forme de cône tronqué. En partant d'un motif triangulaire, on peut obtenir une cavité en forme de pyramide tronquée, etc. Ces techniques de gravure permettent également d'ajuster l'angle des parois de la cavité si on le souhaite.

On comprendra que l'invention n'est, pas limitée aux modes de réalisation particuliers qui ont été décrits ci-dessus ni à un quelconque procédé de fabrication.

L'invention est définie par la revendication 1 ci-dessous.

**Revendications**

1. Implant pour la stimulation électrique d'une structure nerveuse, comprenant:

   - un substrat électriquement isolant (1);
   - un réseau de cavités (2) formées dans une surface supérieure du substrat, les cavités ayant une profondeur supérieure à 15 μm;
   - des électrodes de stimulation (3), chacune disposée au fond de l'une des cavités; et
   - une couche électriquement conductrice formant un plan de masse (4) en partie supérieure des cavités.

2. Implant selon la revendication 1, dans lequel chaque cavité (2) a une forme évasée s'élargissant du fond de la cavité vers la surface supérieure du substrat (1).

3. Implant selon l'une quelconque des revendications précédentes, dans lequel les cavités (2) ont une profondeur inférieure à 50 μm.

4. Implant selon l'une quelconque des revendications précédentes, dans lequel les cavités (2) ont une profondeur comprise entre 25 et 35 μm.

5. Implant selon l'une quelconque des revendications précédentes, dans lequel chaque électrode de stimulation (3) a, sur une surface de fond de sa cavité respective (2), une dimension inférieure à 60 μm.

6. Implant selon l'une quelconque des revendications précédentes, dans lequel chaque cavité (2) a une surface de fond isolante recouverte de manière partielle par une électrode de stimulation (3).

7. Implant selon l'une quelconque des revendications 1 à 5, dans lequel chaque électrode de stimulation (3) a une partie centrale s'étendant sur une surface de fond de sa cavité respective (2) et une partie périphérique (8) débordant sur les parois latérales de ladite cavité.

8. Implant selon la revendication 7, dans lequel ladite partie centrale de l'électrode de stimulation (3) a une dimension inférieure à 40 μm.

9. Implant selon l'une quelconque des revendications précédentes, dans lequel la couche électriquement conductrice (4) formant le plan de masse comporte, pour chaque cavité (2) du réseau, une partie (7) débordant sur les parois latérales de ladite cavité.

**Patentansprüche**

1. Implantat zur elektrischen Stimulation einer Nervenstruktur, umfassend:

   - ein elektrisch isolierendes Substrat (1);
   - ein Netz von Hohlräumen (2), welche in einer oberen Fläche des Substrats gebildet sind, wobei die Hohlräume eine Tiefe größer als 15 μm aufweisen;
   - Stimulationselektroden (3), welche jeweils am Boden von einem der Hohlräume angeordnet sind; und
   - eine elektrisch leitfähige Schicht, welche eine Masseplatte (4) im oberen Teil der Hohlräume bildet.

2. Implantat nach Anspruch 1, wobei jeder Hohlraum (2) eine sich aufweitende Form aufweist, welche sich vom Boden des Hohlraums in Richtung der oberen Fläche des Substrats (1) verbreitert.

3. Implantat nach einem der vorhergehenden Ansprüche, wobei die Hohlräume (2) eine Tiefe kleiner als 50 μm aufweisen.

4. Implantat nach einem der vorhergehenden Ansprüche, wobei die Hohlräume (2) eine Tiefe aufweisen, welche zwischen 25 und 35 μm liegt.

5. Implantat nach einem der vorhergehenden Ansprüche, wobei jede Stimulationselektrode (3) an einer Bodenfläche ihres jeweiligen Hohlraums (2) eine Größe kleiner als 60 μm aufweist.

**6.** Implantat nach einem der vorhergehenden Ansprüche, wobei jeder Hohlraum (2) eine isolierende Bodenfläche aufweist, welche teilweise von einer Stimulationselektrode (3) bedeckt ist.

**7.** Implantat nach einem der Ansprüche 1 bis 5, wobei jede Stimulationselektrode (3) einen Mittelteil, welcher sich an einer Bodenfläche ihres jeweiligen Hohlraums (2) erstreckt, und einen Umfangsteil (8) aufweist, welcher auf die Seitenwände des Hohlraums ragt.

**8.** Implantat nach Anspruch 7, wobei der Mittelteil der Stimulationselektrode (3) eine Größe kleiner als 40 $\mu$m aufweist.

**9.** Implantat nach einem der vorhergehenden Ansprüche, wobei die die Masseplatte bildende elektrisch leitfähige Schicht (4) für jeden Hohlraum (2) des Netzes einen Teil (7) umfasst, welcher auf die Seitenwände des Hohlraums ragt.

**Claims**

**1.** Implant for electrical stimulation of a nerve structure comprising:

- an electrically insulating substrate (1);
- a array of cavities (2) formed in an upper surface of the substrate, the cavities having a depth greater than 15 $\mu$m;
- stimulation electrodes (3), each arranged at a bottom portion of one of the cavities; and
- an electrically conductive layer forming a ground plane (4) at an upper portion of the cavities.

**2.** Implant according to claim 1, wherein each cavity (2) has a flared shape that expands from the bottom portion of the cavity toward the upper surface of the substrate (1).

**3.** Implant according to any one of the preceding claims, wherein the cavities (2) have a depth lower than 50 $\mu$m.

**4.** Implant according to any one of the preceding claims, wherein the cavities (2) have a depth of between 25 and 35 $\mu$m.

**5.** Implant according to any one of the preceding claims, wherein each stimulation electrode (3) has, on a bottom surface of its respective cavity (2), a dimension of less than 60 $\mu$m.

**6.** Implant according to any one of the preceding claims, wherein each cavity (2) has an insulating bottom surface partially covered with a stimulation electrode (3).

**7.** Implant according to any one of claims 1 to 5, wherein each stimulation electrode (3) has a central part extending over a bottom surface of its respective cavity (2) and a peripheral part (8) overlapping the lateral walls of said cavity.

**8.** Implant according to claim 7, wherein said central part of the stimulation electrode (3) has a dimension of less than 40 $\mu$m.

**9.** Implant according to any one of the preceding claims, wherein the electrically conductive layer (4) forming the ground plane comprises, for each cavity (2) of the array, a part (7) overlapping the lateral walls of said cavity.

FIG. 1

FIG. 2

FIG. 3

FIG. 4

FIG. 5

FIG. 6

FIG. 7

FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004073547 A

**Littérature non-brevet citée dans la description**

- **S. JOUCLA ; B. YVERT.** Improved Focalization of Electrical Microstimulation Using Microelectrode Arrays: A Modeling Study. *PLoS ONE,* Mars 2009, vol. 4 (3), 4828, www.plosone.org
- **D. PALANKER.** Migration of retinal cells through a perforated membrane: implications for a high-resolution prosthesis. *Investigative Ophthalmology & Visual Science,* Septembre 2004, vol. 45 (9), 3266-3270
- **D. ROUSSEAU.** Microfabrication of new microelectrode arrays equipped with a ground surface configuration for focal neural microstimulation. *Journal of Micromechanics and Microengineering,* Juin 2009, vol. 19 (7), 074010